# EUROPEAN PATENT SPECIFICATION

(11) **EP 0 418 016 B1**
(45) Date of publication and mention of the grant of the patent: **03.05.1995**
(21) Application number: 90309882.0
(22) Date of filing: 10.09.1990
(51) Int. Cl.: C07D 231/44, A01N 43/56, C07D 231/18

(54) **N-phenylpyrazole derivatives as insecticides**
N-Phenylpyrazolderivate als Insektizide
Dérivés de N-phénylpyrazole comme insecticides

(30) Priority: 11.09.1989 GB 8920521
(43) Date of publication of application: 20.03.1991
(73) Proprietor: RHONE-POULENC AGRICULTURE LTD., Ongar, Essex CM5 0HW (GB)
(72) Inventor: Roberts, David Alan, Research Station of, Ongar, Essex CM5 0HW (GB); Hawkins, David William, Research Station of, Ongar, Essex CM5 0HW (GB); Buntain, Ian George, Research Station of, Ongar, Essex CM5 0HW (GB); McGuire, Ross, Research Station of, Ongar, Essex CM5 0HW (GB)
(74) Representative: Bentham, Stephen

(56) References cited:
- EP-A- 0 234 119
- EP-A- 0 295 117
- EP-A- 0 352 944

## Description

This invention relates to N-phenylpyrazole derivatives, to compositions containing them and to the use of N-phenylpyrazole derivatives against arthropod, plant nematode, helminth and protozoan pests.

N-Phenylpyrazole derivatives of the general formula (A) 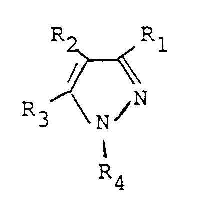
wherein R¹ represents a cyano or nitro group, a halogen, i.e. fluorine, chlorine, bromine or iodine, atom, or an acetyl or formyl group; R² represents a group R⁵SO₂, R⁵SO, or R⁵S in which R⁵ represents a straight or branched chain alkyl, alkenyl or alkynyl group containing up to 4 carbon atoms which may be unsubstituted or substituted by one or more halogen atoms which may be the same or different; R³ represents a hydrogen atom, or an amino group -NR⁶R⁷ wherein R⁶ and R⁷, which may be the same or different, each represent a hydrogen atom or a straight or branched chain alkyl, alkenylalkyl or alkynylalkyl group containing up to 5 carbon atoms, a formyl group, a straight or branched chain alkanoyl group (which contains from 2 to 5 carbon atoms and which may be optionally substituted by one or more halogen atoms) or R⁶ and R⁷ together with the nitrogen atom to which they are attached form a 5 or 6 membered cyclic imide, or represents a straight or branched-chain alkoxycarbonyl group (which contains from 2 to 5 carbon atoms and is unsubstituted or substituted by one or more halogen atoms), or R³ represents a straight or branched-chain alkoxymethyleneamino group containing from 2 to 5 carbon atoms which may be unsubstituted or substituted on methylene by a straight or branched-chain alkyl group containing from 1 to 4 carbon atoms, or represents a halogen, i.e. fluorine, chlorine, bromine or iodine, atom; and R⁴ represents a phenyl group substituted in the 2-position by a fluorine, chlorine, bromine or iodine atom; in the 4-position by a straight or branched chain alkyl or alkoxy group containing from 1 to 4 carbon atoms which may be unsubstituted or substituted by one or more halogen atoms which may be the same or different (the trifluoromethyl and trifluoromethoxy groups are preferred), or a chlorine or bromine atom; and optionally in the 6-position by a fluorine, chlorine, bromine or iodine atom, which have valuable activity against arthropod, plant nematode, helminth and protozoan pests, are among others described in European Patent Publications Nos. 234119 and 295117

The present invention provides N-phenylpyrazole derivatives of the general formula (I) 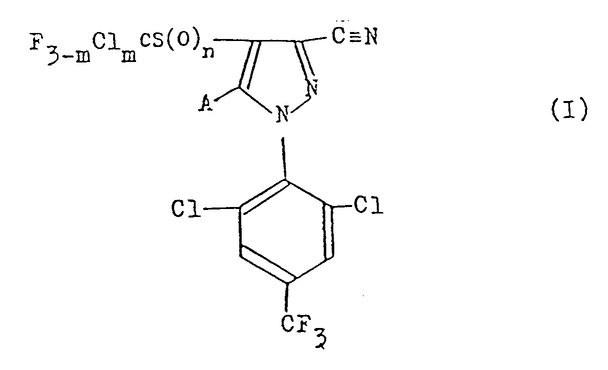
Wherein
A represents a bromine atom or a hydrogen atom
m is the integer 1 or 2
n is Zero or the integer 1 or 2.

The compounds according to the invention have valuable activity against arthropod, plant nematode, helminth and protozoan pests, more particularly by ingestion of the compound(s) of general formula (I) by the arthropods.

Compounds of general formula (I) which are new, processes for their preparation, compositions containing them and methods for their use constitute features of the present invention.

The novel compounds of the invention show improved performance in certain respects, e.g. against arachnids, over the prior art.
Among those compounds one may mention :
1 4-Chlorodifluoromethylthio-3-cyano-1-(2,6-dichloro-4-trifluoromethylphenyl)pyrazole.
2 5-Bromo-4-dichlorofluoromethylthio-3-cyano-1-(2,6-dichloro-4-trifluoromethylphenyl)pyrazole.
3 5-Bromo-4-chlorodifluoromethylthio-3-cyano-1-(2,6-dichloro-4-trifluoromethylphenyl)pyrazole.
4 4-Dichlorofluoromethylthio-3-cyano-1-(2,6-dichloro-4-trifluoromethylphenyi)pyrazole.
5 5-Bromo-4-dichlorofluoromethylsulphinyl-3-cyano-1-(2,6-dichloro-4-trifluoromethylphenyl)pyrazole.
6 5-Bromo-4-dichlorofluoromethylsulphonyl-3-cyano-1-(2,6-dichloro-4-trifluoromethylphenyl)pyrazole.
7 4-Dichlorofluoromethylsulphonyl-3-cyano-1-(2,6-dichloro-4-trifluoromethylphenyl)pyrazole.
8 5-Bromo-4-chlorodifluoromethylsulphinyl-3-cyano-1-(2,6-dichloro-4-trifluoromethylphenyl)pyrazole.
9 4-Dichlorofluoromethylsulphinyl-3-cyano-1-(2,6-dichloro-4-trifluoromethylphenyl)pyrazole.
10 4-chlorodifluoromethylsulphonyl-3-cyano-1-(2,6-dichloro-4-trifluoromethylphenyl)pyrazole
11 4-chlorodifluoromethylsulphinyl-3-cyano-1-(2,6-dichloro-4-trifluoromethylphenyl)pyrazole
In a preferred embodiment m = 2
The numbers 1-11 are assinged to the above compounds for identification and reference hereinafter.

Compounds Nos. 2, 4, 5, 6, 7 and 9 are especially preferred compounds of general formula (I).

In experiments on activity against arthropods carried out on representative compounds, the following results (wherein ppm indicates the concentration of the compound in parts per million of the test solution applied) have been obtained:-

### Test 1

One or more dilutions of the compounds to be tested were made in 50% aqueous acetone.

### (a) Test species: Plutella xylostella (Diamond-back Moth) and Phaedon cochleariae (Mustard Beetle).

Turnip leaf discs were set in agar in petri-dishes and infected with 10 larvae (2nd instar Plutella or 3rd instar Phaedon). Four replicate dishes were assigned to each treatment and were sprayed under a Potter Tower with the appropriate test dilution. After two days live larvae were transferred to similar dishes containing untreated leaves set in agar. Two or three days later the dishes were removed from the constant temperature (25°C) room in which they had been held and the mean percentage mortalities of larvae were determined. These data were corrected against the mortalities in dishes treated with 50% aqueous acetone alone which served as controls.

### (b) Megoura viciae (Vetch Aphid)

Potted tic bean plants previously infected with mixed stages of Megoura were sprayed to run-off using a laboratory turntable sprayer. Treated plants were held in a greenhouse for 2 days and were assessed for aphid mortality using a scoring system, judging the response in comparison with plants treated with 50% aqueous acetone alone, as controls; or by calculating percentage mortality. Each treatment was replicated 4 times.

| | |
|---|---|
| Score 3 | all aphids dead |
| 2 | few aphids alive |
| 1 | most aphids alive |
| 0 | no significant mortality |

### (c) Test species: Spodoptera littoralis.

French bean leaf discs were set in agar in petri-dishes and infected with 5 or 10 larvae (2nd instar). Four replicate dishes were assigned to each treatment and were sprayed under a Potter Tower with the appropriate test dilution. After 2 days live larvae were transferred to similar dishes containing untreated leaves set in agar. Two or three days later the dishes were removed from the constant temperature (25°C) room in which they had been held and the mean percentage mortalities of larvae were determined. These data were corrected against the mortalities in dishes treated with 50% aqueous acetone alone which served as controls.

### (d) Tetranychus urticae (Red spider Mite)

French bean leaf discs previously infected with mixed stages of Tetranychus) were sprayed under the Potter Tower with the appropriate test dilution. Treated discs were kept on sponges over a water bath (30°C) for 2 days and were assessed for mite mortality using a scoring system, judging the response in comparison with discs treated with 50% aqueous acetone alone, as controls. Each treatment was replicated 4 times.

| | |
|---|---|
| Score 3 | all mites dead |
| 2 | few mites alive |
| 1 | most mites alive |
| 0 | no significant mortality |

According to the above method an application of the following compounds was effective against the larvae of Plutella xylostella producing at least 65% mortality at less than or equal to 500ppm: 1 - 11

According to the above method an application of the following compounds was effective against all stages of Megoura viciae producing a score of at least 7/12 at less than or equal to 50ppm or at least 90% mortality at less than or equal to 50ppm: 1 - 11

According to the above method an application of the following compounds was effective against the larvae of Phaedon cochleariae producing at least 90% mortality at less than or equal to 5ppm: 1 - 11

According to the above method an application of the following compounds was effective against the larvae of Spodoptera littoralis producing at least 70% mortality at less than or equal to 500ppm: 1 - 11

According to the above mentioned an application of the following compounds was effective against all active stages of Tetranychus urticae producing a score of at least 9/12 at less than or equal to 500 ppm: 1, 4, 5, 6, 7, 9, 10, 11.

As a result in a fourth preferred embodiement compounds 1, 4, 5, 6, 7, 9, 10, 11 are advantageously used for controlling mites.

According to a feature of the present invention, there is provided a method for the control of arthropod pests at a locus which comprises the treatment of the locus (e.g. by application or administration) with an effective amount of a compound of general formula (I), wherein the various symbols are as hereinbefore defined. The compounds of general formula (I) may, in particular, be used in the field of veterinary medicine and livestock husbandry and in the maintenance of public health against arthropods which are parasitic internally or externally upon vertebrates, particularly warm-blooded vertebrates, for example man and domestic animals, e.g. cattle, sheep, goats, equines, swine, poultry, dogs, cats and fishes, for example Acarina, including ticks (e.g. Ixodes spp., Boophilus spp. e.g. Boophilus microplus, Amblyomma spp., Hyalomma spp., Rhipicephalus spp. e.g. Rhipicephalus appendiculatus, Haemaphysalis spp., Dermacentor spp., Ornithodorus spp. (e.g. Ornithodorus moubata and mites (e.g. Damalinia spp., Dermahyssus gallinae, Sarcoptes spp. e.g. Sarcoptes scabiei, Psoroptes spp., Chorioptes spp., Demodex spp., Eutrombicula spp.,); Diptera (e.g. Aedes spp., Anopheles spp., Musca spp., Hypoderma spp., Gasterophilus spp., Simulium spp.); Hemiptera (e.g. Triatoma spp.); Phthiraptera (e.g. Damalinia spp., Linognathus spp.); Siphonaptera (e.g. Ctenocephalides spp.); Dictyoptera (e.g. Periplaneta spp., Blatella spp.); Hymenoptera (e.g. Monomorium pharaonis); in the protection of stored products, for example cereals, including grain and flour, groundnuts, animal feedstuffs, timber and household goods, e.g. carpets and textiles, against attack by arthropods, more especially beetles, including weevils, moths and mites, for example Ephestia spp. (flour moths), Anthrenus spp. (carpet beetles), Tribolium spp. (flour beetles), Sitophilus spp. (grain weevils) and Acarus spp. (mites), in the control of cockroaches, ants and termites and similar arthropod pests in infested domestic and industrial premises and in the control of mosquito larvae in waterways, wells, reservoirs or other running or standing water; for the treatment of foundations, structure and soil in the prevention of the attack on buildings by termites, for example, Reticulitermes spp., Heterotermes spp., Coptotermes spp.; in agriculture, against adults, larvae and eggs of Lepidoptera (butterflies and moths), e.g. Heliothis spp. such as Heliothis virescens (tobacco budworm), Heliothis armigera and Heliothis zea, Spodoptera spp. such as S.exempta, S.littoralis (Egyptian cotton worm), S.eridania (southern army worm), Mamestra configurata (bertha army worm); Earias spp. e.g. E.insulana (Egyptian bollworm), Pectinophora spp. e.g. Pectinophora gossypiella (pink bollworm), Ostrinia spp. such as O.nubilalis (European cornborer), Trichoplusia ni (cabbage looper), Pieris spp. (cabbage worms), Laphygma spp. (army worms), Agrotis and Amathes spp. (cutworms), Wiseana spp. (porina moth), Chilo spp. (rice stem borer), Tryporyza spp. and Diatraea spp. (sugar cane borers and rice borers), Sparganothis pilleriana (grape berry moth), Cydia pomonella (codling moth), Archips spp. (fruit tree tortrix moths), Plutella xylostella (diamond back moth); against adult and larvae of Coleoptera (beetles) e.g. Hypothenemus hampei (coffee berry borer), Hylesinus spp. (bark beetles), Anthonomus grandis (cotton boll weevil), Acalymma spp. (cucumber beetles), Lema spp., Psylliodes spp., Leptinotarsa decemlineata (Colorado potato beetle), Diabrotica spp. (corn rootworms), Gonocephalum spp. (false wire worms), Agriotes spp. (wireworms), Dermolepida and Heteronychus spp. (white grubs), Phaedon cochleariae (mustard beetle), Lissorhoptrus oryzophilus (rice water weevil), Meligethes spp. (pollen beetles), Ceutorhynchus spp., Rhynchophorus and Cosmopolites spp. (root weevils); against Hemiptera e.g. Psylla spp., Bemisia spp., Trialeurodes spp., Aphis spp., Myzus spp., Megoura viciae, Phylloxera spp., Adelges spp., Phorodon humuli (hop damson aphid), Aeneolamia spp., Nephotettix spp. (rice leaf hoppers), Empoasca spp., Nilaparvata spp., Perkinsiella spp., Pyrilla spp., Aonidiella spp. (red scales), Coccus spp., Pseucoccus spp., Helopeltis spp. (mosquito bugs), Lygus spp., Dysdercus spp., Oxycarenus spp., Nezara spp.; Hymenoptera e.g. Athalia spp. and Cephus spp. (saw flies), Atta spp. (leaf cutting ants); Diptera e.g. Hylemyia spp. (root flies), Atherigona spp. and Chlorops spp. (shoot flies), Phytomyza spp. (leaf miners), Ceratitis spp. (fruit flies); Thysanoptera such as Thrips tabaci; Orthoptera such as Locusta and Schistocerca spp. (locusts) and crickets e.g. Gryllus spp. and Acheta spp.; Collembola e.g. Sminthurus spp. and Onychiurus spp. (springtails), Isoptera e.g. Odontotermes spp. (termites), Dermaptera e.g. Forficula spp. (earwigs) and also other arthropods of agricultural significance such as Acari (mites) e.g. Tetranychus spp., Panonychus spp. and Bryobia spp. (spider mites), Eriophyes app. (gall mites), Polyphagotarsonemus spp.; Blaniulus spp. (millipedes), Scutigerella spp. (symphilids), Oniscus spp. (woodlice) and Triops spp. (crustacea);
The invention also provides a method for the control of arthropod pests of plants which comprises the application to the plants or to the medium in which they grow of an effective amount of a compound of general formula (I).

For the control of arthropods, the active compound is generally applied to the locus in which arthropod infestation is to be controlled at a rate of about 0.1kg to about 25kg of active compound per hectare of locus treated. Under ideal conditions, depending on the pest to be controlled, the lower rate may offer adequate protection. On the other hand, adverse weather conditions, resistance of the pest and other factors may require that the active ingredient be used in higher proportions. In foliar application, a rate of 1g to 1000g/ha may be used.

When the pest is soil-borne, the formulation containing the active compound is distributed evenly over the area to be treated in any convenient manner. Application may be made, if desired, to the field or crop-growing area generally or in close proximity to the seed or plant to be protected from attack. The active component can be washed into the soil by spraying with water over the area or can be left to the natural action of rainfall. During or after application, the formulation can, if desired, be distributed mechanically in the soil, for example by ploughing or disking. Application can be prior to planting, at planting, after planting but before sprouting has taken place or after sprouting.

The compounds of general formula (I) are of value in controlling pests which feed on parts of the plant remote from the point of application, e.g. leaf feeding insects are killed by the subject compounds applied to roots.

In addition the compounds may reduce attacks on the plant by means of antifeeding or repellent effects.

The compounds of general formula (I) are of particular value in the protection of field, forage, plantation, glasshouse, orchard and vineyard crops, of ornamentals and of plantation and forest trees, for example, cereals (such as maize, wheat, rice, sorghum), cotton, tobacco, vegetables and salads (such as beans, cole crops, curcurbits, lettuce, onions, tomatoes and peppers), field crops (such as potato, sugar beet, ground nuts, soyabean, oil seed rape), sugar cane, grassland and forage (such as maize, sorghum, lucerne), plantations (such as of tea, coffee, cocoa, banana, oil palm, coconut, rubber, spices), orchards and groves (such as of stone and pip fruit, citrus, kiwifruit, avocado, mango, olives and walnuts), vineyards, ornamental plants, flowers and shrubs under glass and in gardens and parks, forest trees (both deciduous and evergreen) in forests, plantations and nurseries.

They are also valuable in the protection of timber (standing, felled, converted, stored or structural) from attack by sawflies (e.g. Urocerus) or beetles (e.g. scolytids, platypodids, lyctids, bostrychids, cerambycids, anobiids), or termites, for example, Reticulitermes spp., Heterotermes spp., Coptotermes spp.

They have applications in the protection of stored products such as grains, fruits, nuts, spices and tobacco, whether whole, milled or compounded into products, from moth, beetle and mite attack. Also protected are stored animal products such as skins, hair, wool and feathers in natural or converted form (e.g. as carpets or textiles) from moth and beetle attack; also stored meat and fish from beetle, mite and fly attack.

The compounds of general formula (I) are of particular value in the control of arthropods, which are injurious to, or spread or act as vectors of diseases in man and domestic animals, for example those hereinbefore mentioned, and more especially in the control of ticks, mites, lice, fleas, midges and biting, nuisance and myiasis flies. The compounds of general formula (I) are particularly useful in controlling arthropods, which are present inside domestic host animals or which feed in or on the skin or suck the blood of the animal, for which purpose they may be administered orally, parenterally, percutaneously or topically.

The compositions hereinafter described for topical application to man and animals and in the protection of stored products, household goods, property and areas of the general environment may, in general, alternatively be employed for application to growing crops and crop growing loci and as a seed dressing.

Suitable means of applying the compounds of general formula (I) include:-
to persons or animals infested by or exposed to infestation by arthropods, by parenteral, oral or topical application of compositions in which the active ingredient exhibits an immediate and/or prolonged action over a period of time against the arthropods, for example by incorporation in feed or suitable orally-ingestible pharmaceutical formulations, edible baits, salt licks, dietary supplements, pour-on formulations, sprays, baths, dips, showers, jets, dusts, greases, shampoos, creams, wax-smears and livestock self-treatment systems; to the environment in general or to specific locations where pests may lurk, including stored products, timber, household goods, and domestic and industrial premises, as sprays, fogs, dusts, smokes, wax-smears, lacquers, granules and baits, and in tricklefeeds to waterways, wells, reservoirs and other running or standing water; to domestic animals in feed to control fly larvae feeding in their faeces;
to growing crops as foliar sprays, dusts, granules, fogs and foams; also as suspensions of finely divided and encapsulated compounds of general formula (I); as soil and root treatments by liquid drenches, dusts, granules, smokes and foams; and as seed dressings by liquid slurries and dusts.

The compounds of general formula (I) may be applied to control arthropods in compositions of any type known to the art suitable for internal or external administration to vertebrates or application for the control of arthropods in any premises or indoor or outdoor area, containing as active ingredient at least one compound of general formula (I) in association with one or more compatible diluents or adjuvants appropriate for the intended use. All such compositions may be prepared in any manner known to the art.

Compositions suitable for administration to vertebrates or man include preparations suitable for oral, parenteral, percutaneous, e.g. pour-on, or topical administration.

Compositions for oral administration comprise one or more of the compounds of general formula (I) in association with pharmaceutically acceptable carriers or coatings and include, for example, tablets, pills, capsules, pastes, gels, drenches, medicated feeds, medicated drinking water, medicated dietary supplements, slow-release boluses or other slow-release devices intended to be retained within the gastro-intestinal tract. Any of these may incorporate active ingredient contained within microcapsules or coated with acid-labile or alkali-labile or other pharmaceutically acceptable enteric coatings. Feed premixes and concentrates containing compounds of the present invention for use in preparation of medicated diets, drinking water or other materials for consumption by animals may also be used.

Compositions for parenteral administration include solutions, emulsions or suspensions in any suitable pharmaceutically acceptable vehicle and solid or semisolid subcutaneous implants or pellets designed to release active ingredient over a protracted period and may be prepared and made sterile in any appropriate manner known to the art.

Compositions for percutaneous and topical administration include sprays, dusts, baths, dips, showers, jets, greases, shampoos, creams, wax-smears, or pour-on preparations and devices (e.g. ear tags) attached externally to animals in such a way as to provide local or systemic arthropod control.

Solid or liquid baits suitable for controlling arthropods comprise one or more compounds of general formula (I) and a carrier or diluent which may include a food substance or some other substance to induce comsumption by the arthropod.

Liquid compositions include water miscible concentrates, emulsifiable concentrates, flowable suspensions, wettable or soluble powders containing one or more compounds of general formula (I) which may be used to treat substrates or sites infested or liable to infestation by arthropods including premises, outdoor or indoor storage or processing areas, containers or equipment and standing or running water.

Solid homogenous or heterogenous compositions containing one or more compounds of general formula (I), for example granules, pellets, briquettes or capsules, may be used to treat standing or running water over a period of time. A similar effect may be achieved using trickle or intermittent feeds of water dispersible concentrates as described herein.

Compositions in the form of aerosols and aqueous or non-aqueous solutions or dispersions suitable for spraying, fogging and low- or ultra-low volume spraying may also be used.

Suitable solid diluents which may be used in the preparation of compositions suitable for applying the compounds of general formula (I) include aluminium silicate, kieselguhr, corn husks, tricalcium phosphate, powdered cork, absorbent carbon black, magnesium silicate, a clay such as kaolin, bentonite or attapulgite, and water soluble polymers and such solid compositions may, if desired, contain one or more compatible wetting, dispersing, emulsifying or colouring agents which, when solid, may also serve as diluent.

Such solid compositions, which may take the form of dusts, granules or wettable powders, are generally prepared by impregnating the solid diluents with solutions of the compound of general formula (I) in volatile solvents, evaporating the solvents and, if necessary, grinding the products so as to obtain powders and, if desired, granulating or compacting the products so as to obtain granules, pellets or briquettes or by encapsulating finely divided active ingredient in natural or synthetic polymers, e.g. gelatin, synthetic resins and polyamides.

The wetting, dispersing and emulsifying agents which may be present, particularly in wettable powders, may be of the ionic or non-ionic types, for example sulphoricinoleates, quaternary ammonium derivatives or products based upon condensates of ethylene oxide with nonyl- and octyl-phenol, or carboxylic acid esters of anhydrosorbitols which have been rendered soluble by etherification of the free hydroxy groups by condensation with ethylene oxide, or mixtures of these types of agents. Wettable powders may be treated with water immediately before use to give suspensions ready for application.

Liquid compositions for the application of the compounds of general formula (I) may take the form of solutions, suspensions and emulsions of the compounds of general formula (I) optionally encapsulated in natural or synthetic polymers, and may, if desired, incorporate wetting, dispersing or emulsifying agents. These emulsions, suspensions and solutions may be prepared using aqueous, organic or aqueous-organic diluents, for example acetophenone, isophorone, toluene, xylene, mineral, animal or vegetable oils, and water soluble polymers (and mixtures of these diluents), which may contain wetting, dispersing or emulsifying agents of the ionic or non-ionic types or mixtures thereof, for example those of the types described above. When desired, the emulsions containing the compounds of general formula (I) may be used in the form of self-emulsifying concentrates containing the active substance dissolved in the emulsifying agents or in solvents containing emulsifying agents compatible with the active substance, the simple addition of water to such concentrates producing compositions ready for use.

Compositions containing compounds of general formula (I) which may be applied to control arthropod pests, may also contain synergists (e.g. piperonyl butoxide or sesamex), stabilizing substances, other insecticides, acaricides, plant nematocides, anthelmintics or anticoccidials, fungicides (agricultural or veterinary as apropriate e.g. benomyl, iprodione), bactericides, arthropod or vertebrate attractants or repellents or pheromones, reodorants, flavouring agents, dyes and auxiliary therapeutic agents, e.g. trace elements. These may be designed to improve potency, persistence, safety, uptake where desired, spectrum of pests controlled or to enable the composition to perform other useful functions in the same animal or area treated.

Examples of other pesticidally-active compounds which may be included in, or used in conjunction with, the compositions of the present invention are:-acephate, chlorpyrifos, demeton-S-methyl, disulfoton, ethoprofos, fenitrothion, malathion, monocrotophos, parathion, phosalone, pirimiphos-methyl, triazophos, cyfluthrin, cypermethrin, deltamethrin, fenpropathrin, fenvalerate, permethrin, aldicarb, carbosulfan, methomyl, oxamyl, pirimicarb, bendiocarb, teflubenzuron, dicofol, endosulfan, lindane, benzoximate, cartap, cyhexatin, tetradifon, avermectins, ivermectin, milbemycins, thiophanate, trichlorfon, dichlorvos, diaveridine and dimetridazole.

The compositions for application to control arthropod pests usually contain from 0.00001% to 95%, more particularly from 0.0005% to 50%, by weight of one or more compounds of general formula (I) or of total active ingredients (that is to say the compound(s) of general formula (I) together with other substances toxic to arthropods and optionally plant nematodes, anthelmintics, anticoccidials, synergists, trace elements or stabilisers). The actual compositions employed and their rate of application will be selected to achieve the desired effect(s) by the farmer, livestock producer, medical or veterinary practitioner, pest control operator or other person skilled in the art. Solid and liquid compositions for application topically to animals, timber, stored products or household goods usually contain from 0.00005% to 90%, more particularly from 0.001% to 10%, by weight of one or more compounds of general formula (I). For administration to animals orally or parenterally, including percutaneously solid and liquid compositions normally contain from 0.1% to 90% by weight of one or more compound of general formula (I). Medicated feedstuffs normally contain from 0.001% to 3% by weight of one or more compounds of general formula (I). Concentrates and supplements for mixing with feedstuffs normally contain from 5% to 90%, and preferably from 5% to 50%, by weight of one or more compounds of general formula (I). Mineral salt licks normally contain from 0.1% to 10% by weight of one or more compounds of general formula (I).

Dusts and liquid compositions for application to livestock, persons, goods, premises or outdoor areas may contain 0.0001% to 15%, and more especially 0.005% to 2.0%, by weight of one or more compounds of general formula (I). Suitable concentrations in treated waters are between 0.0001 ppm are 20 ppm, and more especially 0.001 ppm to 5.0 ppm. of one or more compounds of general formula (I) and may also be used therapeutically in fish farming with appropriate exposure times. Edible baits may contain from 0.01% to 5% and preferably 0.01% to 1.0%, by weight of one or more compounds of general formula (I).

When administered to vertebrates parenterally, orally or by percutaneous or other means, the dosage of compounds of general formula (I) will depend upon the species, age and health of the vertebrate and upon the nature and degree of its actual or potential infestation by arthropod pest. A single dose of 0.1 to 100 mg, preferably 2.0 to 20.0 mg, per kg body weight of the animal or doses of 0.01 to 20.0 mg, preferably 0.1 to 5.0 mg, per kg body weight of the animal per day for sustained medication are generally suitable by oral or parenteral administration. By use of sustained release formulations or devices, the daily doses required over a period of months may be combined and administered to animals on a single occasion.

The following Composition Examples illustrate compositions for use against arthropod pests which comprise, as active ingredient, compounds of general formula (I). The compositions described in Composition Examples 1 to 6 can each be diluted in water to give a sprayable composition at concentrations suitable for use in the field.

### COMPOSITION EXAMPLE 1

A water soluble concentrate was prepared from

| | |
|---|---|
| Compound No. 6 | 7% w/v |
| Ethylan BCP | 10% w/v |
| and N-methylpyrrolidone | to 100% by volume |

by dissolving the Ethylan BCP in a portion of N-methylpyrrolidone, and then adding the active ingredient with heating and stirring until dissolved. The resulting solution was made up to volume by adding the remainder of the solvent.

### COMPOSITION EXAMPLE 2

An emulsifiable concentrate was prepared from

| | |
|---|---|
| Compound No. 6 | 7% w/v |
| Soprophor BSU | 4% w/v |
| Arylan CA | 4% w/v |
| N-methylpyrrolidone | 50% w/v |
| and Solvesso 150 | to 100% by volume |

by dissolving Soprophor BSU, Arylan CA and the active ingredient in N-methylpyrrolidone, and then adding Solvesso 150 to volume.

### COMPOSITION EXAMPLE 3

A wettable powder was prepared from

| | |
|---|---|
| Compound No. 6 | 40% w/w |
| Arylan S | 2% w/w |
| Darvan No. 2 | 5% w/w |
| and Celite PF | to 100% by weight |

by mixing the ingredients, and grinding the mixture in a hammer-mill to a particle size less than 50 microns.

### COMPOSITION EXAMPLE 4

An aqueous flowable formulation was prepared from

| | |
|---|---|
| Compound No. 6 | 30% w/v |
| Ethylan BCP | 1% w/v |
| Sopropon T36 | 0.2% w/v |
| Ethylene glycol | 5% w/v |
| Rhodigel 23 | 0.15% w/v |
| and Water | to 100% by volume |

by intimately mixing the ingredients and grinding in a bead mill until the median particle size was less than 3 microns.

### COMPOSITION EXAMPLE 5

An emulsifiable suspension concentrate was prepared

| | |
|---|---|
| from Compound No. 6 | 30% w/v |
| Ethylan BCP | 10% w/v |
| Bentone 38 | 0.5% w/v |
| and Solvesso 150 | to 100% by volume |

by intimately mixing the ingredients and grinding in a bead mill until the median particle size was less than 3 microns.

### COMPOSITION EXAMPLE 6

Water dispersible granules were prepared from

| | |
|---|---|
| Compound No. 6 | 30% w/w |
| Darvan No. 2 | 15% w/w |
| Arylan S | 8% w/w |
| and Celite PF | to 100% by weight |

by mixing the ingredients, micronising in a fluid-energy mill, and then granulating in a rotating pelletiser by spraying on sufficient water (up to 10% w/w). The resulting granules were dried in a fluid-bed drier to remove excess water.
Descriptions of commercial ingredients used in the foregoing Composition Examples:-
- Ethylan BCP: nonylphenol ethylene oxide condensate
- Soprophor BSU: condensate of tristyrylphenol and ethylene oxide
- Arylan CA: 70% w/v solution of calcium dodecylbenzenesulphonate
- Solvesso 150: light C₁₀-aromatic solvent
- Arylan S: sodium dodecylbenzenesulphonate
- Darvan: sodium lignosulphonate
- Celite PF: synthetic magnesium silicate carrier
- Sopropon T36: sodium salt of polycarboxylic acid
- Rhodigel 23: polysaccharide xanthan gum
- Bentone 38: organic derivative of magnesium montmorillonite

### COMPOSITION EXAMPLE 7

A dusting powder may be prepared by intimately mixing:-

| | |
|---|---|
| Compound No. 6 | 1 to 10% w/w (weight/weight) |
| Talc superfine | to 100% by weight |

This powder may be applied to a locus of arthropod infestation, for example refuse tips or dumps, stored products or household goods or animals infested by, or at risk of infestation by, arthropods to control the arthropods by oral ingestion. Suitable means for distributing the dusting powder to the locus of arthropod infestation include mechanical blowers, handshakers and livestock self treatment devices.

### COMPOSITION EXAMPLE 8

An edible bait may be prepared by intimately mixing:-

| | |
|---|---|
| Compound No. 6 | 0.1 to 1.0% w/w |
| Wheat flour | 80% w/w |
| Molasses | to 100% w/w |

This edible bait may be distributed at a locus, for example domestic and industrial premises, e.g. kitchens, hospitals or stores, or outdoor areas, infested by arthropods, for example ants, locusts, cockroaches and flies, to control the arthropods by oral ingestion.

### COMPOSITION EXAMPLE 9

A solution may be prepared containing:-

| | |
|---|---|
| Compound No. 6 | 15% w/v (weight/volume) |
| Dimethylsulphoxide | to 100% by volume |

by dissolving the pyrazole derivative in a portion of the dimethyl-sulphoxide and then adding more dimethylsulphoxide to the desired volume. This solution may be applied to domestic animals infested by arthropods, percutaneously as a pour-on application or, after sterilisation by filtration through a polytetrafluoroethylene membrane (0.22 micrometre pore size), by parenteral injection, at a rate of application of from 1.2 to 12 ml of solution per 100 kg of animal body weight.

### COMPOSITION EXAMPLE 10

A wettable powder may be formed from:-

| | |
|---|---|
| Compound No. 6 | 50% w/w |
| Ethylan BCP (a nonylphenol/ethylene oxide condensate containing 9 moles of ethylene oxide per mol of phenol) | 5% w/w |
| Aerosil (silicon dioxide of microfine-particle size) | 5% w/w |
| Celite PF (synthetic magnesium silicate carrier) | 40% w/w |

by adsorbing the Ethylan BCP onto the Aerosil, mixing with the other ingredients and grinding the mixture in a hammer-mill to give a wettable powder, which may be diluted with water to a concentration of from 0.001% to 2% w/v of the pyrazole compound and applied to a locus of infestation by arthropods, for example dipterous larvae, by spraying, or to domestic animals infested by, or at risk of infestation by, arthropods by spraying or dipping, or by oral administration as drinking water, to control the arthropods.

### COMPOSITION EXAMPLE 11

A slow release bolus may be formed from granules containing a density agent, binder, slow-release agent and Compound No. 6 at varying percentage compositions. By compressing the mixture a bolus with a specific gravity of 2 or more can be formed and may be administered orally to ruminant domestic animals for retention within the reticulo-rumen to give a continual slow release of pyrazole compound over an extended period of time to control infestation of the ruminant domestic animals by arthropods.

### COMPOSITION EXAMPLE 12

A slow release composition may be prepared from:-

| | |
|---|---|
| Compound No. 6 | 0.5 to 25% w/w |
| polyvinylchloride base | to 100% w/w |

by blending the polyvinylchloride base with the pyrazole compound and a suitable plasticiser, e.g. dioctyl phthalate, and melt-extruding or hot-moulding the homogenous composition into suitable shapes, e.g. granules, pellets, brickettes or strips, suitable, for example, for addition to standing water or, in the case of strips, fabrication into collars or ear-tags for attachment to domestic animals, to control insect pests by slow release of the pyrazole compound.

Similar compositions may be prepared by replacing the compound of general formula (I) in the Composition Examples by the appropriate quantity of any other compound of the invention.

The compounds of general formula (I) can be prepared by the application or adaptation of known methods (i.e. methods heretofore used or described in the chemical literature), generally pyrazole ring formation followed where necessary by changing substituents, for example as described in European Patent Publications Nos. 234119 and 295117 (and United States Patent Applications Serial Nos. 943132 and 205238).

It is to be understood that in the description of the following processes that the sequences for the introduction of the various groups on the pyrazole ring may be performed in a different order and that suitable protecting groups may be required as will be apparent to those skilled in the art: compounds of general formula (I) may be converted by known methods into other compounds of general formula (I).

According to a process of the present invention, compounds of general formula (I) in which n is the integer 1 or 2 may be prepared by oxidation of the sulphur atoms of the corresponding thio compounds of formula (I) wherein n is zero; the oxidation may be effected employing oxidants of the formula:-

R¹⁰-O-O-H (II)

wherein R¹⁰ represents the hydrogen atom, or a trifluoroacetyl or 3-chlorobenzoyl group in a solvent e.g. dichloromethane or chloroform dichloroethane or trifluoroacetic acid and at temperatures from 0°C to 100°C, or with a reagent such as potassium hydrogen persulphate or potassium salt of Caro's acid in a solvent e.g. methanol and water, and at a temperature from -30°C to 50°C.

According to a further process, compounds of general formula (I) wherein A represents a bromine atom may be prepared by the diazotisation of the corresponding compound of general formula (I) in which A represents the amino group using sodium nitrite in a mineral acid, for example a mixture of concentrated sulphuric acid and acetic acid, at a temperature from 0° to 60°C, and by subsequent reaction with a copper salt and a mineral acid at a temperature from 0° to 100°C. The diazotisation may alternatively be performed employing an alkyl nitrite e.g. tert-butyl nitrite in the presence of a suitable halogenating agent preferably bromoform at temperatures from 0°C to 100°C, and optionally in the presence of an inert solvent, preferably acetonitrile or chloroform.

According to a further process, compounds of general formula (I) wherein A represents a hydrogen atom may be prepared by treatment of a compound of general formula (I) wherein A represents an amino group, with a diazotising agent preferably tertiary butyl nitrite in a solvent, preferably tetrahydrofuran, and at ambient to the reflux temperature. 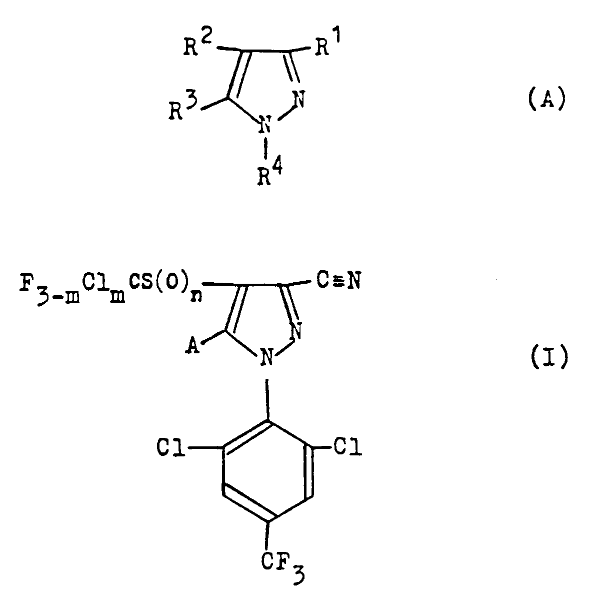
The following Examples and Reference Examples illustrate the preparation of compounds of general formula (I) according to the present invention: [Chromatography was effected on a silica column (May & Baker Laboratory Products 40/60 flash silica) at a pressure of 6.8Nm⁻², unless otherwise stated.]

### REFERENCE EXAMPLE 1

A solution of 5-amino-4-chlorodifluoromethylthio-3-cyano-1-(2,6-dichloro-4-trifluoromethylphenyl)pyrazole (0.9g) in trifluoroacetic acid(5ml) was cooled to 0°C and treated with a solution of 30% aqueous hydrogen peroxide (0.27ml) in trifluoroacetic acid (0.5ml). The solution was maintained at 0°C for 4h and then at approximately 6°C overnight (18h). The solution was poured into water (20ml) and extracted into dichloromethane (2x15ml). The combined dichloromethane extracts were washed with aqueous sodium metabisulphite solution (20ml), aqueous sodium bicarbonate solutioh (20ml), water (20ml), 2 molar hydrochloric acid (20ml)and water (20ml), then dried (magnesium sulphate), filtered and evaporated to yield a yellow solid (0.69g). Purification by chromatography (eluant dichloromethane: hexane 5:1) gave 5-amino-4-chlorodifluoromethylsulphinyl-3-cyano-1-(2,6-dichloro-4-trifluoromethylphenyl)pyrazole (0.4g), m.p. 203-205°C, in the form of a white solid.

By proceeding in a similar manner, but using the appropriate starting material there were prepared:-5-amino-4-dichlorofluoromethylsulphinyl-3-cyano-1-(2,6-dichloro-4-trifluoromethylphenyl)pyrazole, m.p. 206-207°C, in the form of a colourless solid. 4-chlorodifluoromethylsulphinyl-3-cyano-1-(2,6-dichloro-4-trifluoromethylphenyl) pyrazole, m.p. 114-116°C, in the form of a brown solide.

### REFERENCE EXAMPLE 1

A solution of 5-amino-3-cyano-1-(2,6-dichloro-4-trifluoromethylphenyl)pyrazole (20.0g) in dichloromethane (100ml) was stirred magnetically and treated dropwise with a solution of trifluoromethylsulphenyl chloride (10.8g) in dichloromethane (50ml) during 1 hour. The solution was stirred overnight at room temperature, then washed with water (100ml), dried over anhydrous magnesium sulphate, filtered, and evaporated in vacuo to give a solid (26.3g). This solid was recrystallised from toluene/hexane to give 5-amino-3-cyano-1-(2,6-dichloro-4-trifluoromethylphenyl)-4-trifluoromethylthiopyrazole as fawn crystals (24.2g) m.p. 169-171°C.
By proceeding in a similar manner but replacing the 5-amino-3-cyano-1-(2,6-dichloro-4-trifluoromethylphenyl)pyrazole by the hereinafter indicated appropriately substituted pyrazole there was obtained from trifluoromethylsulphenyl chloride unless otherwise stated:
5-Amino-3-cyano-1-(2,6-dichloro-4-trifluoromethoxyphenyl)-4-trifluoro-methylthiopyrazole, m.p. 125-126°C, in the form of pale yellow crystals, from 5-amino-3-cyano-1-(2,6-dichloro-4-trifluoromethoxyphenyl)pyrazole.
5-Amino-3-cyano-1-(2,6-dichloro-4-difluoromethoxyphenyl)-4-trifluoromethylthiopyrazole, m.p. 127-128.5°C, in the form of a buff solid, from 5-amino-3-cyano-1-(2,6-dichloro-4-difluoromethoxyphenyl)pyrazole.
5-Amino-1-(2-chloro-4-trifluoromethylphenyl)-3-cyano-4-trifluoromethylthiopyrazole, m.p. 142-144°C, in the form of a light brown solid, from 5-amino-1-(2-chloro-4-trifluoromethylphenyl)-3-cyanopyrazole.
5-Amino-3-cyano-1-(2,4,6-trichlorophenyl)-4-trifluoromethylthiopyrazole, m.p. 192-193°C, in the form of brown crystals, from 5-amino-3-cyano-1-(2,4,6-trichlorophenyl)pyrazole.
5-Amino-3-cyano-1-(2,6-di-bromo-4-trifluoromethylphenyl-4-trifluoromethylthiopyrazole, m.p. 202-204°C, in the form of orange crystals, from 5-amino-3-cyano-1-(2,6-dibromo-4-trifluoromethylphenyl)pyrazole.
5-Amino-1-(2-bromo-4-trifluoromethylphenyl)-3-cyano-4-trifluoromethylthiopyrazole, m.p. 136-138°C, in the form of a pale yellow solid, from 5-amino-1-(2-bromo-4-trifluoromethylphenyl)-3-cyanopyrazole.
5-Amino-3-cyano-1-(2,6-dichloro-4-trifluoromethylphenyl)-4-difluoromethylthiopyrazole, m.p. 159-161°C, in the form of a light brown solid, from 5-amino-3-cyano-1-(2,6-dichloro-4-trifluoromethylphenyl)pyrazole and difluoromethylsulphenyl chloride.
5-Amino-3-cyano-1-(2,6-dichloro-4-trifluoromethylphenyl)-4-heptafluoropropylthiopyrazole, m.p. 148-150°C, in the form of a yellow solid, after dry column flash chromatography on silica eluting with dichloromethane and petroleum ether (2:1); from 5-amino-3-cyano-1-(2,6-dichloro-4-trifluoromethylphenyl) pyrazole and heptafluoropropylsulphenyl chloride.
5-Amino-1-(2-bromo-6-chloro-4-trifluoromethylphenyl)-3-cyano-4-trifluoromethylthiopyrazole, m.p. 183-185°C, in the form of yellow crystals, from 5-amino-1-(2-bromo-6-chloro-4-trifluoromethylphenyl)-3-cyanopyrazole and employing tetrahydrofuran as solvent.
5-Amino-3-cyano-1-(2,6-dichloro-4-trifluoromethylphenyl)-4-trichloromethylthiopyrazole, m.p. 245-247°C, in the form of a white solid after purification by chromatography, starting from 5-amino-3-cyano-1-(2,6-dichloro-4-trifluoromethylphenyl)pyrazole and trichloromethylsulphenyl chloride.
By proceeding in a similar manner but replacing the trifluoromethylsulphenyl chloride by dichlorofluoromethylsulphenyl chloride, and by the addition of a molar equivalent of pyridine to the reaction mixture after stirring overnight there was obtained:
5-Amino-3-cyano-4-dichlorofluoromethylthio-1-(2,6-dichloro-4-trifluoromethylphenyl)pyrazole, m.p. 178-180°C in the form of a white solid, after purification by chromatography eluting with diethyl ether/hexane (1:1); from 5-amino-3-cyano-1-(2,6-dichloro-4-trifluoromethylphenyl)pyrazole.
By proceeding in a similar manner but employing a molar equivalent of pyridine in the reaction solution there was obtained:
5-Amino-3-chloro-1-(2,6-dichloro-4-trifluoromethylphenyl)-4-trifluoromethylthiopyrazole, m.p. 149-150.5°C, in the form of a white solid, after recrystallisation from hexane from 5-amino-3-chloro-1-(2,6-dichloro-4-trifluoromethylphenyl)pyrazole.
5-Amino-3-bromo-1-(2,6-dichloro-4-trifluoromethylphenyl)-4-trifluoromethylthiopyrazole, m.p. 154.5-156°C, in the form of a white solid, after recrystallisation from hexane/ethyl acetate and then from hexane/cyclohexane, starting from 5-amino-3-bromo-1-(2,6-dichloro-4-trifluoromethylphenyl)pyrazole.
5-Amino-1-(2,6-dichloro-4-trifluoromethylphenyl)-3-fluoro-4-trifluoromethylthiopyrazole, m.p. 123-126°C, in the form of a white solid, starting from 5-amino-1-(2,6-dichloro-4-trifluoromethylphenyl)-3-fluoropyrazole.
5-Amino-4-chlorodifluoromethylthio-3-cyano-1-(2,6-dichloro-4-trifluoromethylphenyl)pyrazole, m.p. 167-168°C, in the form of a white solid, starting from chlorodifluoromethylsulphenyl chloride. The product was purified by high performance liquid chromatography employing a 8 micron irregular column (21.4mm x 25cm) and eluting with acetonitrile/water (3:2).

### EXAMPLE 2

### Compounds Nos. 6 and 7

A solution of 5-amino-4-dichlorofluoromethylthio-3-cyano-1-(2,6-dichloro-4-trifluoromethylphenyl)pyrazole (3.0g) was stirred in a mixture of bromoform (10ml) and dry acetonitrile (10ml). Tert-butyl nitrite (2.04g) was added dropwise during 5 minutes, and the mixture heated at 60-70°C for 4 hours. Evaporation in vacuo gave a brown solid which was purified by dry column flash chromatography eluting with dichloromethane/hexane (1:1) to give after recrystallisation from toluene/petroleum ether 5-bromo-4-dichlorofluoromethylthio-3-cyano-1-(2,6-dichloro-4-trifluoromethylphenyl)pyrazole as a pale yellow solid (1.81g), m.p. 162-163.5°C.

By proceeding in a similar manner but using the appropriate starting material there was prepared:-
5-bromo-4-chlorodifluoromethylthio-3-cyano-1-(2,6-dichloro-4-trifluoromethylphenyl)pyrazole as an off-white solid, m.p. 143-146°C, from dry column flash chromatography eluting with dichloromethane/hexane (2:1).

### EXAMPLE 3

### Compounds Nos. 9, 5 and 14

A solution of 5-amino-4-dichlorofluoromethylthio-3-cyano-1-(2,6-dichloro-4-trifluoromethylphenyl)pyrazole (3.55g) in dry tetrahydrofuran (30ml) was added to tert-butyl nitrite (4.83g) at room temperature. The mixture was stirred at room temperature for 3 days and evaporated in vacuo to give a brown oil. Purification by dry column flash chromatography eluting with hexane/ dichloromethane (1:2) gave 4-dichlorofluoromethylthio-3-cyano-1-(2,6-dichloro-4-trifluoromethylphenyl)pyrazole (1.98g),m.p. 132-133°C, in the form of a pale yellow solid, after recrystallisation from toluene/petroleum ether.
By proceeding in a similar manner but using the appropriate starting materials there were prepared:-
4-chlorodifluoromethylthio-3-cyano-1-(2,6-dichloro-4-trifluoromethylphenyl)pyrazole, m.p. 127-130°C, in the form of a colourless solid, using an additional quantity of tert-butyl nitrite (3 equivalents) and beating under reflux for 4 hours; 4-dichlorofluoromethylsulphinyl-3-cyano-1-(2,6-dichloro-4-trifluoromethylphenyl)pyrazole, m.p. 110-112°C, in the form of a colourless solid, after heating under reflux for 3 hours.

### Reference EXAMPLE 5

A partial solution of 5-amino-4-chlorodifluoromethylthio-3-cyano-1-(2,6-dichloro-4-trifluoromethylphenyl)-pyrazole (0.9g) in dichloromethane (20ml) was stirred and treated with m-chloroperbenzoic acid (1.0g). After 20 hours at room temperature the mixture was heated under reflux for 6 hours. The cooled mixture was washed with a solution of sodium metabisulphite (1 x 20ml), then with sodium hydroxide solution (2 x 10ml) and finally with water (21 x 10ml). The organic layer was dried over anhydrous magnesium sulphate, filtered, and evaporated in vacuo to give a solid. Purification by dry column flash chromatography on silica, eluting with dichloromethane/hexane(5:1) gave 5-amino-4-chlorodifluoromethylsulphonyl-3-cyano-1-(2,6-dichloro-4-trifluoromethylphenyl)pyrazole as an off-white solid (0.23g), m.p. 210-211.5°C.
By proceeding in a similar manner from the appropriate starting material but reacting at room temperature for 61 hours there was prepared:-
5-amino-4-dichlorofluoromethylsulphonyl-3-cyano-1-(2,6-dichloro-4-trifluoromethylphenyl)pyrazole as a pale yellow solid, m.p. 224-225.5°C.

### EXAMPLE 6

### Compounds Nos 11, 10, 12, 13 and 15

A stirred mixture of 85% hydrogen peroxide (0.65g) and chloroform (15ml) was treated with trifluoroacetic anhydride (3.33g) in chloroform (10ml) stirred at 0°C. After 15 minutes the mixture was allowed to reach room temperature, and a solution of 5-bromo-4-dichlorofluoromethylthio-3-cyano-1-(2,6-dichloro-4-trifluoromethylphenyl)pyrazole (1.4g) in chloroform (20ml) was added, and the solution stirred for 24 hours, then poured onto water (50ml), and the dichloromethane layer washed in turn with 5% sodium dithionite solution (10ml), sodium carbonate solution (10ml) and with water (20ml). This solution was then dried over anhydrous magnesium sulphate, and evaporated in vacuo to give a solid, which was purified by chromatography eluting with hexane/ diethyl ether (4:1) to give 5-bromo-4-dichlorofluoromethylsulphonyl-3-cyano-1-(2,6-dichloro-4-trifluoromethylphenyl)pyrazole (0.37g), m.p. 160-162°C, in the form of a white solid after recrystallisation from toluene/petroleum ether. Further elution with the same solvent gave 5-bromo-4-dichlorofluoromethylsulphinyl-3-cyano-1-(2,6-dichloro-4-trifluoromethylphenyl)pyrazole (0.15g), m.p. 126-129°C, in the form of a white solid after recrystallisation from toluene/petroleum ether.
By proceeding in a similar manner, but using the appropriate starting material there were prepared:-
4-dichlorofluoromethylsulphonyl-3-cyano-1-(2,6-dichloro-4-trifluoromethylphenyl)pyrazole, m.p. 161-163°C, in the form of a colourless solid after reaction in dichloroethane for 2 days at room temperature followed by 6 hours at reflux;
5-bromo-4-chlorodifluoromethylsulphinyl-3-cyano-1-(2,6-dichloro-4-trifluoromethylphenyl)pyrazole, m.p. 149-152°C, in the form of a colourless solid after reaction in dichloromethane for 3 hours.
4-chlorodifluoromethylsulphonyl-3-cyano-1-(2,6-dichloro-4-trifluoromethylphenyl) pyrazole, m.p. 157-158°C, in the form of a white solid after reaction in dichloroethane at ambient temperature overnight then heating at 50°C for two hours.

## Claims (Claims for the following Contracting State(s): AT, BE, CH, LI, DE, DK, FR, GB, GR, IT, LU, NL, SE)

1. An N-phenylpyrazole derivative of the general formula: 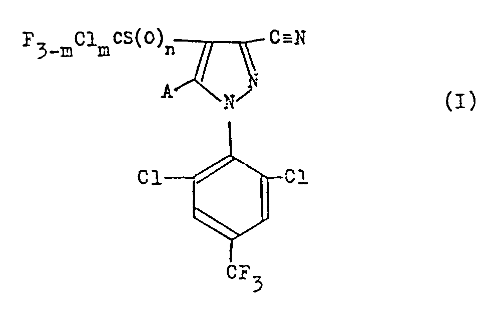 wherein
A represents a bromine or hydrogen atom;
m is the integer 1 or 2; and
n is zero or the integer 1 or 2.

2. A compound according to claim 1 wherein m = 2.

3. A compound according to claim 1 which is
5-Bromo-4-dichlorofluoromethylthio-3-cyano-1-(2,6-dichloro-4-trifluoromethylphenyl)pyrazole,
4-Dichlorofluoromethylthio-3-cyano-1-(2,6-dichloro-4-trifluoromethylphenyl)pyrazole,
5-Bromo-4-dichlorofluoromethylsulphinyl-3-cyano-1-(2,6-dichloro-4-trifluoromethylphenyl)pyrazole,
5-Bromo-4-dichlorofluoromethylsulphonyl-3-cyano-1-(2,6-dichloro-4-trifluoromethylphenyl)pyrazole,
4-Dichlorofluoromethylsulphonyl-3-cyano-1-(2,6-dichloro-4-trifluoromethylphenyl)pyrazole, or
4-Dichlorofluoromethylsulphinyl-3-cyano-1-(2,6-dichloro-4-trifluoromethylphenyl)pyrazole.

4. A process for the preparation of an N-phenylpyrazole derivative according to claim 1 which comprises:
(A) when n is the integer 1 or 2 the oxidation of the corresponding thio compound of general formula (I) wherein n is zero;
(B) when A represents a bromine atom by the diazotisation of the corresponding compound of general formula (I) wherein A represents the amino group and replacement of the diazotised amino group by bromine;
(C) when A represents a hydrogen atom by the conversion to hydrogen of the amino group of the corresponding compound of general formula (I) wherein A represents the amino group.

5. An arthropodicidal composition which comprises a compound according to any one of claims 1 to 3 and one or more compatible diluents or carriers.

6. A method for the control of arthropod pests at a locus which comprises treatment of the locus with an effective amount of compound according to any one of claims 1 to 3.

7. A method according to claim 6 wherein the arthropods are mites.

8. A method for the control of arthropods according to claim 7 wherein the compound is
4-Chlorodifluoromethylthio-3-cyano-1-(2,6-dichloro-4-trifluoromethylphenyl)pyrazole,
4-dichlorofluoromethylthio-3-cyano-1-(2,6-dichloro-4-trifluoromethylphenyl)pyrazole ,
5-Bromo-4-dichlorofluoromethylsulphonyl-3-cyano-1-(2,6-dichloro-4-trifluoromethylphenyl)pyrazole,
4-Dichlorofluoromethylsulphonyl-3-cyano-1-(2,6-dichloro-4-trifluoromethylphenyl)pyrazole,
4-Dichlorofluoromethylsulphinyl-3-cyano-1-(2,6-dichloro-4-trifluoromethylphenyl)pyrazole,
4-chlorodifluoromethylsulphonyl-3-cyano-1-(2,6-dichloro-4-trifluoromethylphenyl)pyrazole or
4-chlorodifluoromethylsulphenyl-3-cyano-1-(2,6-dichloro-4-trifluoromethylphenyl)pyrazole.

## Claims (Claims for the following Contracting State(s): ES)

1. A process for the preparation of an N-phenylpyrazole derivative of the general formula: 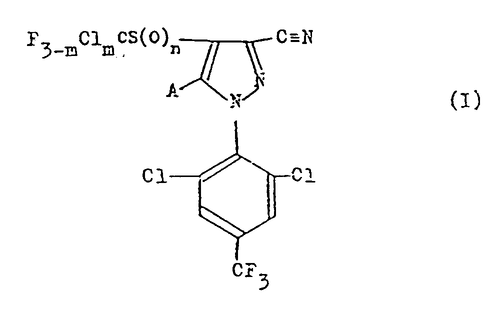 wherein
A represents a bromine or hydrogen atom;
m is the integer 1 or 2; and
n is zero or the integer 1 or 2.
(A) when n is the integer 1 or 2 the oxidation of the corresponding thio compound of general formula (I) wherein n is zero;
(B) when A represents a bromine atom by the diazotisation of the corresponding compound of general formula (I) wherein A represents the amino group and replacement of the diazotised amino group by bromine;
(C) when A represents a hydrogen atom by the conversion to hydrogen of the amino group of the corresponding compound of general formula (I) wherein A represents the amino group.

2. A process according to claim 1 wherein m = 2.

3. A process according to claim 1 in which the derivative of formula I is 5-Bromo-4-dichlorofluoromethylthio-3-cyano-1-(2,6 dichloro-4-trifluoromethylphenyl)pyrazole,
4-Dichlorofluoromethylthio-3-cyano-1-(2,6-dichloro-4-trifluoromethylphenyl)pyrazole,
5-Bromo-4-dichlorofluoromethylsulphinyl-3-cyano-1-(2,6-dichloro-4-trifluoromethylphenyl)pyrazole,
5-Bromo-4-dichlorofluoromethylsulphonyl-3-cyano-1-(2,6-dichloro-4-trifluoromethylphenyl)pyrazole,
4-Dichlorofluoromethylsulphonyl-3-cyano-1-(2,6-dichloro-4-trifluoromethylphenyl)pyrazole, or
4-Dichlorofluoromethylsulphinyl-3-cyano-1-(2,6-dichloro-4-trifluoromethylphenyl)pyrazole.

4. A method for the control of arthropod pests at a locus which comprises treatment of the locus with an effective amount of compound prepared according to any one of claims 1 to 3.

5. A method according to claim 4 wherein the arthropods are mites.

6. A method for the control of arthropods according to claim 5 wherein the compound is
4-Chlorodifluoromethylthio-3-cyano-1-(2,6-dichloro-4-trifluoromethylphenyl)pyrazole,
4-dichlorofluoromethylthio-3-cyano-1-(2,6-dichloro-4-trifluoromethylphenyl)pyrazole ,
5-Bromo-4-dichlorofluoromethylsulphonyl-3-cyano-1-(2,6-dichloro-4-trifluoromethylphenyl)pyrazole,
4-Dichlorofluoromethylsulphonyl-3-cyano-1-(2,6-dichloro-4-trifluoromethylphenyl)pyrazole,
4-Dichlorofluoromethylsulphinyl-3-cyano-1-(2,6-dichloro-4-trifluoromethylphenyl)pyrazole,
4-chlorodifluoromethylsulphonyl-3-cyano-1-(2,6-dichloro-4-trifluoromethylphenyl)pyrazole or
4-chlorodifluoromethylsulphenyl-3-cyano-1-(2,6-dichloro-4-trifluoromethylphenyl)pyrazole.

## Patentansprüche (Patentansprüche für folgende(n) Vertragsstaat(en): AT, BE, CH, LI, DE, DK, FR, GB, GR, IT, LU, NL, SE)

1. N-Phenylpyrazolderivate der allgemeinen Formel 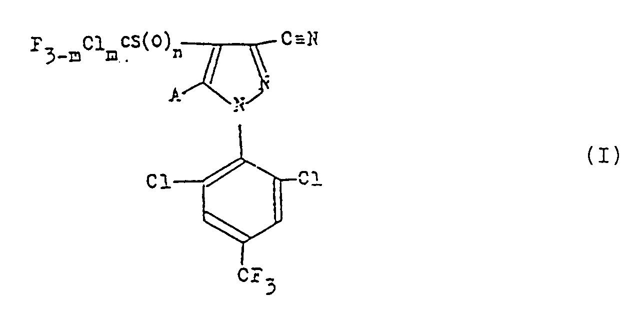 worin
A ein Brom- oder Wasserstoffatom bedeutet,
m eine ganze Zahl 1 oder 2 und
n Null oder eine ganze Zahl 1 oder 2 ist.

2. Verbindungen gemäß Anspruch 1, worin m 2 ist.

3. Verbindungen gemäß Anspruch 1, wobei die Verbindungen die folgenden sind:
5-Brom-4-dichlorfluormethylthio-3-cyano-1-(2,6-dichlor-4-trifluormethylphenyl)-pyrazol;
4-Dichlordifluormethylthio-3-cyano-1-(2,6-dichlor-4-trifluormethylphenyl)-pyrazol;
5-Brom-4-dichlorfluormethylsylfinyl-3-cyano-1-(2,6-dichlor-4-trifluormethylphenyl)-pyrazol;
5-Brom-4-dichlorfluormethylsylfonyl-3-cyano-1-(2,6-dichlor-4-trifluormethylphenyl)-pyrazol;
4-Dichlorfluormethylsylfonyl-3-cyano-1-(2,6-dichlor-4-trifluormethylphenyl)-pyrazol oder
4-Dichlorfluormethylsulfinyl-3-cyano-1-(2,6-dichlor-4-trifluormethylphenyl)-pyrazol.

4. Verfahren zur Herstellung von N-Phenylpyrazolderivaten gemäß Anspruch 1, das umfaßt:
(A) Wenn n eine ganze Zahl von 1 oder 2 ist, Oxidation der entsprechenden Thioverbindung der allgemeinen Formel (I), worin n Null bedeutet;
(B) wenn A ein Bromatom bedeutet, Diazotieren der entsprechenden Verbindung der allgemeinen Formel (I), worin A eine Aminogruppe bedeutet, und Ersetzen der diazotierten Aminogruppe durch Brom;
(C) wenn A ein Wasserstoffatom bedeutet, Umwandlung der Aminogruppe der entsprechenden Verbindung der allgemeinen Formel (I), worin A eine Aminogruppe bedeutet, in Wasserstoff.

5. Arthropodicide Zusammensetzungen, die eine Verbindung gemäß einem der Ansprüche 1 bis 3 und eine oder mehrere verträgliche Verdünnungsmittel oder Träger enthalten.

6. Verfahren zur lokalen Kontrolle eines Befalls durch Arthropoden, das die Behandlung des Ortes mit einer wirksamen Menge einer Verbindung gemäß einem der Ansprüche 1 bis 3 umfaßt.

7. Verfahren gemaß Anspruch 6, wobei die Arthropoden Milben sind.

8. Verfahren zur Kontrolle von Artbropoden gemäß Anspruch 7, wobei die Verbindungen die folgenden sind:
4-Chlordifluormethylthio-3-cyano-1-(2,6-dichlor-4-trifluormethylphenyl)-pyrazol,
4-Dichlorfluormethylthio-3-cyano-1-(2,6-dichlor-4-trifluormethylphenyl)-pyrazol,
5-Brom-4-dichlorfluormethylsulfonyl-3-cyano-1-(2,6-dichlor-4-trifluormethylphenyl)-pyrazol,
4-Dichlorfluormethylsulfonyl-3-cyano-1-(2,6-dichlor-4-trifluormethylphenyl)-pyrazol,
4-Dichlorfluormethylsulfinyl-3-cyano-1-(2,6-dichlor-4-trifluormethylphenyl)-pyrazol,
4-Chlordifluormethylsulfonyl-3-cyano-1-(2,6-dichlor-4-trifluormethylphenyl)-pyrazol oder
4-Chlordifluormethylsulfenyl-3-cyano-1-(2,6-dichlor-4-trifluormethylphenyl)-pyrazol.

## Patentansprüche (Patentansprüche für folgende(n) Vertragsstaat(en): ES)

1. Verfahren zur Herstellung von N-Phenylpyrazolderivaten der allgemeinen Formel 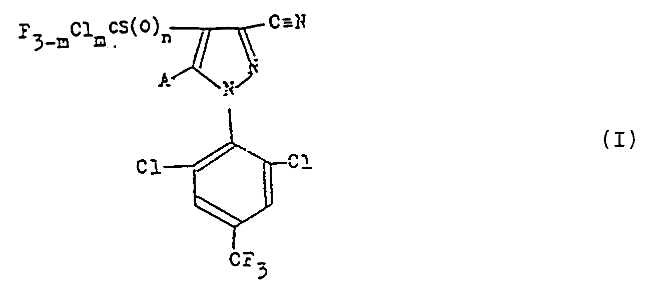 worin
A ein Brom- oder Wasserstoffatom bedeutet,
m eine ganze Zahl 1 oder 2 und
n Null oder eine ganze Zahl 1 oder 2 ist;
(A) Wenn n eine ganze Zahl von 1 oder 2 ist, Oxidation der entsprechenden Thioverbindung der allgemeinen Formel (I), worin n Null bedeutet;
(B) wenn A ein Bromatom bedeutet, Diazotieren der entsprechenden Verbindung der allgemeinen Formel (I), worin A eine Aminogruppe bedeutet, und Ersetzen der diazotierten Aminogruppe durch Brom;
(C) wenn A ein Wasserstoffatom bedeutet, Umwandlung der Aminogruppe der entsprechenden Verbindung der allgemeinen Formel (I), worin A eine Aminogruppe bedeutet, in Wasserstoff.

2. Verfahren gemäß Anspruch 1, worin m 2 ist.

3. Verfahren gemäß Anspruch 1, wobei die Derivate der Formel (I) die folgenden sind:
5-Brom-4-dichlorfluormethylthio-3-cyano-1-(2,6-dichlor-4-trifluormethylphenyl)-pyrazol;
4-Dichlorfluormethylthio-3-cyano-1-(2,6-dichlor-4-trifluormethylphenyl)-pyrazol;
5-Brom-4-dichlorfluormethylsylfinyl-3-cyano-1-(2,6-dichlor-4-trifluormethylphenyl)-pyrazol;
5-Brom-4-dichlorfluormethylsylfonyl-3-cyano-1-(2,6-dichlor-4-trifluormethylphenyl)-pyrazol;
4-Dichlorfluormethylsylfonyl-3-cyano-1-(2,6-dichlor-4-trifluormethylphenyl)-pyrazol oder
4-Dichlorfluormethylsulfinyl-3-cyano-1-(2,6-dichlor-4-trifluormethylphenyl)-pyrazol.

4. Verfahren zur lokalen Kontrolle eines Befalls durch Arthropoden, das die Behandlung des Ortes mit einer wirksamen Menge einer gemäß einem der Ansprüche 1 bis 3 hergestellten Verbindung umfaßt.

5. Verfahren gemäß Anspruch 4, wobei die Arthropoden Milben sind.

6. Verfahren zur Kontrolle von Artbropoden gemäß Anspruch 5, wobei die Verbindungen die folgenden sind:
4-Chlordifluormethylthio-3-cyano-1-(2,6-dichlor-4-trifluormethylphenyl)-pyrazol,
4-Dichlorfluormethylthio-3-cyano-1-(2,6-dichlor-4-trifluormethylphenyl)-pyrazol,
5-Brom-4-dichlorfluormethylsulfonyl-3-cyano-1-(2,6-dichlor-4-trifluormethylphenyl)-pyrazol,
4-Dichlorfluormethylsulfonyl-3-cyano-1-(2,6-dichlor-4-trifluormethylphenyl)-pyrazol,
4-Dichlorfluormethylsulfinyl-3-cyano-1-(2,6-dichlor-4-trifluormethylphenyl)-pyrazol,
4-Chlordifluormethylsulfonyl-3-cyano-1-(2,6-dichlor-4-trifluormethylphenyl)-pyrazol oder
4-Chlordifluormethylsulfenyl-3-cyano-1-(2,6-dichlor-4-trifluormethylphenyl)-pyrazol.

## Revendications (Revendications pour l'(les) Etat(s) contractant(s) suivant(s): AT, BE, CH, LI, DE, DK, FR, GB, GR, IT, LU, NL, SE)

1. Dérivé de N-phénylpyrazole de formule générale : 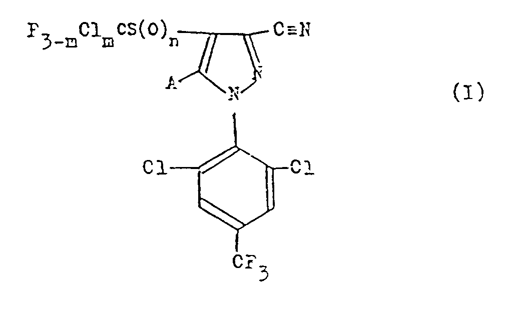 dans laquelle
A représente un atome de brome ou d'hydrogène ;
m représente le nombre entier 1 ou 2 ; et
n est égal à zéro ou au nombre entier 1 ou 2.

2. Composé suivant la revendication 1, dans lequel m est égal à 2.

3. Composé suivant la revendication 1, qui est
le 5-bromo-4-dichlorofluorométhylthio-3-cyano-1-(2,6-dichloro-4-trifluorométhylphényl)pyrazole,
le 4-dichlorofluorométhylthio-3-cyano-1-(2,6-dichloro-4-trifluorométhylphényl)pyrazole,
le 5-bromo-4-dichlorofluorométhylsulfinyl-3-cynao-1-(2,6-dichloro-4-trifluorométhylphényl)pyrazole,
le 5-bromo-4-dichlorofluorométhylsulfonyl-3-cyano-1-(2,6-dichloro-4-trifluorométhylphényl)pyrazole,
le 4-dichlorofluorométhylsulfonyl-3-cyano-1-(2,6-dichloro-4-trifluorométhylphényl)pyrazole, ou
le 4-dichlorofluorométhylsulfinyl-3-cyano-1-(2,6-dichloro-4-trifluorométhylphényl)pyrazole.

4. Procédé de préparation d'un dérivé de N-phénylpyrazole suivant la revendication 1, qui comprend :
(A) lorsque n est le nombre entier 1 ou 2, l'oxydation du composé à fonction thio correspondant, de formule générale (I) dans laquelle n est égal à zéro ;
(B) lorsque A représente un atome de brome, la diazotation du composé correspondant de formule générale (I) dans laquelle A représente un groupe amino et le remplacement du groupe amino diazoté par le brome ;
(C) lorsque A représente un atome d'hydrogène, le remplacement par l'hydrogène du groupe amino du composé correspondant de formule générale (I) dans laquelle A représente le groupe amino.

5. Composition destinée à lutter contre des arthropodes qui comprend un composé suivant l'une quelconque des revendications 1 à 3 et un ou plusieurs diluants ou supports compatibles.

6. Procédé de lutte contre des arthropodes parasites dans un milieu, qui comprend le traitement de ce milieu avec une quantité efficace d'un composé suivant l'une quelconque des revendications 1 à 3.

7. Procédé suivant la revendication 6, dans lequel les arthropodes sont des acariens.

8. Procédé de lutte contre des arthropodes suivant la revendication 7, dans lequel le composé est
le 4-chlorodifluorométhylthio-3-cyano-1-(2,6-dichloro-4-trifluorométhylphényl)pyrazole,
le 4-dichlorofluorométhylthio-3-cyano-1-(2,6-dichloro-4-trifluorométhylphényl)pyrazole,
le 5-bromo-4-dichlorofluorométhylsulfonyl-3-cyano-1-(2,6-dichloro-4-trifluorométhylphényl)pyrazole,
le 4-dichlorofluorométhylsulfonyl-3-cyano-1-(2,6-dichloro-4-trifluorométhylphényl)pyrazole,
le 4-dichlorofluorométhylsulfinyl-3-cyano-1-(2,6-dichloro-4-trifluorométhylphényl)pyrazole,
le 4-chlorodifluorométhylsulfonyl-3-cyano-1-(2,6-dichloro-4-trifluorométhylphényl)pyrazole, ou
le 4-chlorodifluorométhylsulfényl-3-cyano-1-(2,6-dichloro-4-trifluorométhylphényl)pyrazole.

## Revendications (Revendications pour l'(les) Etat(s) contractant(s) suivant(s): ES)

1. Procédé de préparation d'un dérivé de N-phénylpyrazole de formule générale : 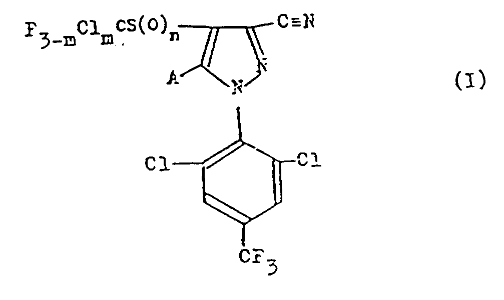 dans laquelle
A représente un atome de brome ou d'hydrogène ;
m est le nombre entier 1 ou 2 ; et
n est égal à zéro ou au nombre entier 1 ou 2.
(A) lorsque n représente le nombre entier 1 ou 2, par oxydation du composé à fonction thio correspondant, de formule générale (I) dans laquelle n est égal à zéro ;
(B) lorsque A représente un atome de brome, par diazotation du composé correspondant de formule générale (I) dans laquelle A représente un groupe amino et le remplacement du groupe amino diazoté par le brome ;
(C) lorsque A représente un atome d'hydrogène, par remplacement par l'hydrogène du groupe amino du composé correspondant de formule générale (I) dans laquelle A représente le groupe amino.

2. Procédé suivant la revendication 1, dans lequel m est égal à 2.

3. Procédé suivant la revendication 1, dans lequel le dérivé de formule I est
le 5-bromo-4-dichlorofluorométhylthio-3-cyano-1-(2,6-dichloro-4-trifluorométhylphényl)pyrazole,
le 4-dichlorofluorométhylthio-3-cyano-1-(2,6-dichloro-4-trifluorométhylphényl)pyrazole,
le 5-bromo-4-dichlorofluorométhylsulfinyl-3-cynao-1-(2,6-dichloro-4-trifluorométhylphényl)pyrazole,
le 5-bromo-4-dichlorofluorométhylsulfonyl-3-cyano-1-(2,6-dichloro-4-trifluorométhylphényl)pyrazole,
le 4-dichlorofluorométhylsulfonyl-3-cyano-1-(2,6-dichloro-4-trifluorométhylphényl)pyrazole, ou
le 4-dichlorofluorométhylsulfinyl-3-cyano-1-(2,6-dichloro-4-trifluorométhylphényl)pyrazole.

4. Procédé pour lutter contre des arthropodes parasites dans un milieu, qui comprend le traitement de ce milieu avec une quantité efficace d'un composé préparé suivant l'une quelconque des revendications 1 à 3.

5. Procédé suivant la revendication 4, dans lequel les arthropodes sont des acariens.

6. Procédé de lutte contre des arthropodes suivant la revendication 5, dans lequel le composé est
le 4-chlorodifluorométhylthio-3-cyano-1-(2,6-dichloro-4 trifluorométhylphényl)pyrazole,
le 4-dichlorofluorométhylthio-3-cyano-1-(2,6-dichloro-4-trifluorométhylphényl)pyrazole,
le 5-bromo-4-dichlorofluorométhylsulfonyl-3-cyano-1-(2,6-dichloro-4-trifluorométhylphényl)pyrazole,
le 4-dichlorofluorométhylsulfonyl-3-cyano-1-(2,6-dichloro-4-trifluorométhylphényl)pyrazole,
le 4-dichlorofluorométhylsulfinyl-3-cyano-1-(2,6-dichloro-4-trifluorométhylphényl)pyrazole,
le 4-chlorodifluorométhylsulfonyl-3-cyano-1-(2,6-dichloro-4-trifluorométhylphényl)pyrazole, ou
le 4-chlorodifluorométhylsulfényl-3-cyano-1-(2,6-dichloro-4-trifluorométhylphényl)pyrazole.
